# EUROPEAN PATENT APPLICATION

(11) **EP 2 241 629 A1**
(43) Date of publication of application: **20.10.2010**
(21) Application number: 09704160.2
(22) Date of filing: 21.01.2009
(51) Int. Cl.: C12P 7/42

(54) **PROCESS FOR PRODUCTION OF OPTICALLY ACTIVE ORTHO-SUBSTITUTED MANDELIC ACID COMPOUND**

(30) Priority: 22.01.2008 JP 2008011300
(71) Applicant: Sumitomo Chemical Company, Limited, Tokyo 104-8260 (JP)
(72) Inventor: ASAKO, Hiroyuki, Toyonaka-shi Osaka 561-0802 (JP)
(74) Representative: Vossius & Partner
(86) International application number: PCT/JP2009/051297
(87) International publication number: WO 2009/093745

(57) **Abstract**

The present invention provides a process of asymmetrically reducing an ortho-substituted phenylglyoxalic acid compound to produce a corresponding optically-active ortho-substituted mandelic acid compound by using a microorganism having an ability of reducing the ortho-substituted phenylglyoxalic acid compound into the optically-active ortho-substituted mandelic acid compound, or a treated product thereof.

## Description

### TECHNICAL FIELD

The present invention relates to a process for producing an optically-active ortho-substituted mandelic acid compound, and so on.

### BACKGROUND ART

As for a method for producing an optically-active mandelic acid compound having a substituent at the ortho-position of a phenyl group, it has been known a method of chemically synthesizing it from the corresponding ortho-substituted phenylglyoxalic acid compound with an asymmetric catalyst containing ruthenium (see, for example, Organic Letters (2005), 7 (24), 5425-5427), but the obtained alcohol has not been necessarily satisfactory in optical purity. Also, there has not been known a method of asymmetrically reducing a phenylglyoxalic acid compound having a carbonyl group under sterically-crowded environment two produce the corresponding optically-active mandelic acid compound with a good optical yield .

### DISCLOSURE OF THE INVENTION

The present invention provides a process for producing an optically-active ortho-substituted mandelic acid compound from an ortho-substituted phenylglyoxalic acid compound with a good optical yield.

Specifically, the present invention provides:
1. A process for producing an optically-active ortho-substituted mandelic acid compound represented by the formula (2): 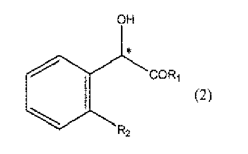 wherein R₁ represents an optionally-substituted amino group or an optionally-substituted alkoxy group, R₂ represents a C1-8 alkyl group optionally substituted with C1-4 alkoxy, and the carbon atom with a symbol * is an asymmetric carbon atom; the process comprising bringing an ortho-substituted phenylglyoxalic acid compound represented by the formula (1): 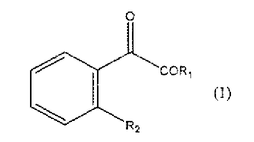 wherein R₁ represents an optionally-substituted amino group or an optionally-substituted alkoxy group, and R₂ represents a C1-8 alkyl group optionally substituted with C1-4 alkoxy; into contact with a microorganism or a treated product thereof, the microorganism having an ability of reducing the compound of the formula (1) into the optically-active ortho-substituted mandelic acid compound of the formula (2) and being selected from the microorganism group consisting of the genus *Aquaspirillum,* the genus *Xanthomonas,* the genus *Curtobacterium,* the genus *Flavobacterium,* the genus *Sphingomonas* and the genus *Stenotrophomonas* (hereinafter sometimes referred to as the present microorganism);
2. The process according to the item 1, wherein the microorganism is *Aquaspirillum itersonii, Xanthomonas campestris, Xanthomonas maltophilia, Curtobacterium albiduz, Curtobacterium citreum, Curtobacterium luteum, Curtobacterium pusillum, Flavobacterium flavescens, Sphingomonas paucimobilis, Sphingomonas parapaucimobilis, Sphingomonas* sp., *Stenotrophomonas rhizophila,* or *Stenotrophomonas* sp.; and
3. The process according to the item 1, wherein the microorganism is *Aquaspirillum itersonii* subsp. *nipponicum* IFO 13615t, *Xanthomonas campestris* IFO 13551, *Xanthomonas maltophilia* JCM 1975t, *Curtobacterium albidum* JCM 1344t, *Curtobacterium citreum* JCM 1345t, *Curtobacterium luteum* JCM 1480t, *Curtobacterium pusillum* JCM 1350t, *Flavobacterium flavescens* JCM 7456, *Sphingomonas paucimobilis* IFO 13935t, *Sphingomonas paucimobilis* JCM 7509, *Sphingomonas paucimobilis* JCM 7511, *Sphingomonas paucimobilis* JCM 7515, *Sphingomonas paucimobilis* JCM 7519, *Sphingomonas parapaucimobilis* JCM 7512, *Sphingomonas parapaucimobilis* JCM 7520, *Sphingomonas* sp. JCM 7513, *Sphingomonas* sp. JCM 7514, *Stenotrophomonas rhizophila* JCM13333, or *Stenotrophomonas* sp. SC-1 (FERM BP-10785); and so on.

According to the present invention, optically-active ortho-substituted mandelic acid compounds can be produced with a good optical yield.

### MODE FOR CARRYING OUT THE INVENTION

In the ortho-substituted phenylglyoxalic acid compound of the formula (1) and in the ortho-substituted mandelic acid compound of the formula (2), a substituent represented by R₁ will be described.

Examples of an optionally-substituted amino group represented by R₁ include, in addition to an amino group, a C1-6 alkylamino group such as a methlyamino group, an methylamino group, a propylamino group, an isopropylamino group, a butylamino group, an isobutylamino group, a t-butylamino group, a pentylamino group, and a hexylamino group. In addition, examples of an optionally-substituted alkoxy group include a C1-8 alkoxy group such as a methoxy group, an ethoxy group, a propoxy group, an isopropoxy group, a butoxy group, a pentyloxy group, a hexyloxy group, a heptyloxy group and an octyloxy group.

Then, a substituent represented by R₂ will be described.

Examples of the C1-8 alkyl group represented by R₂ include a methyl group, an ethyl group, a propyl group, an isopropyl group, a butyl group, a pentyl group, a hexyl group, a heptyl group and an octyl group.

Examples of the C1-8 alkyl group substituted with C1-4 alkoxy include a methoxymethyl group, a methoxyethyl group, a methoxypropyl group, a methoxybutyl group, a methoxypentyl groups, a methoxyhexyl group, a methoxyheptyl group, a methoxyoctyl group, an ethoxymethyl group, an ethoxyethyl group, an ethoxypropyl group, an ethoxybutyl group, an ethoxypentyl group, an ethoxyhexyl group, an ethoxyheptyl group, an ethoxyoctyl group, a propoxymethyl group, a propoxyethyl group, a propoxypropyl group, a propoxybutyl group, a propoxypentyl group, a propoxyhexyl group, a propoxyheptyl group, a propoxyoctyl group, a butoxymethyl group, a butoxyethyl group, a butoxypropyl group, a butoxybutyl group, a butoxypentyl group, a butoxyhexyl group, a butoxyheptyl group and a butoxyoctyl group.

In the compound of the formula (1), as for the optionally-substituted amino group represented by R₁, a methylamino group is preferable, and as for the optionally-substituted alkoxy group, for example, a methoxy group or an ethoxy group is preferable. As for the C1-8 alkyl group represented by R₂, a methyl group is preferable, and as for the C1-8 alkyl group substituted with C1-4 alkoxy, for example, a methoxymethyl group is preferable.

Preferred example of the compound of the formula (1) include 2-(2-methyl-phenyl)-N-methyl-2-oxo-acetamide, ethyl 2-(2-methyl-phenyl)-2-oxoacetate, 2-(2-methoxymethyl-phenyl)-N-methyl-2-oxo-acetamide and ethyl 2-(2-methoxymethyl-phenyl)-2-oxoacetate.

In the method of the present invention, specific examples of the optically active ortho-substituted mandelic acid compound of the formula (2) obtained when the ortho-substituted phenylglyoxalic acid compound described above is a substrate include optically-active substances of 2-(2-methylphenyl)-N-methyl-2-hydroxy-acetomide, ethyl 2-(2-methylphenyl)-2-hydroxyacetate, 2-(2-methoxymethyl-phenyl)-N-methyl-2-hydroxy-acetamide and ethyl 2-(2-methoxymethyl-phenyl)-2-hydroxyacetate.

Cells of the microorganism or treated products thereof used in the production process of the present invention may be cells or treated products thereof of the microorganism having an ability of reducing the ortho-substituted phenylglyoxalic acid compound of the formula (1) into the optically-active ortho-substituted mandelic acid compound of the formula (2), and examples thereof include cells or treated products thereof of microorganisms belonging to the genus *Aquaspirillum* such as *Aquaspirillum itersonii;* microorganisms belonging to the genus *Xanthomonas* such as *Xanthomonas campestris* and *Xanthomonas maltophilia;* microorganisms belonging to the genus *Curtobacterium* such as *Curtobacterium albidum, Curtobacterium citreum, Curtobacterium luteum* and *Curtobacterium pusillum;* microorganisms belonging to the genus *Flavobacterium* such as *Flavobacterium flavescens;* microorganisms belonging to the genus *Sphingomonas* such as *Sphingomonas paucimobilis*, *Sphingomonas parapaucimobilis* and *Sphingomonas* sp.; and microorganisms belonging to the genus *Stenotrophomonas* such as *Stenotrophomonas rhizophila* and *Stenotrophomonas* sp..

More specific examples thereof include cells or treated products thereof of *Aquaspirillum itersonii* subsp. *nipponicum* IFO 13615t, *Xanthomonas campestris* IEO 13551, *Xanthomonas maltophilia* JCM 1975t, *Curtobacterium albidum* JCM 1344t, *Curtobacterium citreum* JCM 1345t, *Curtobacterium luteum* JCM 1480t, *Curtobacterium pusillum* JCM 1350t, *Flavobacterium flavescens* JCM 7456, *Sphingamanas paucimobilis* IFO 13935t, *Sphingomonas paucimobilis* JCM 7509, *Sphingomonas paucimobilis* JCM 7511, *Sphingomonas paucimobilis* JCM 7515, *Sphingomonas paucimobilis* JCM 7519, *Sphingomonas parapaucimobilis* JCM 7512, *Sphingomonas parapaucimobilis* JCM 7520, *Sphingomonas* sp. JCM 7513, *Sphingomonas* sp. JCM 7514, *Stenotrophomonas rhizophila* JCM13333 and *Stenotrophomonas* sp. SC-1 (FERM BP-10785, date of deposit: February 21, 2007).

The *Stenotrophomonas* sp. SC-1 strain was deposited on February 21, 2007, in International Patent Organism Depositary (IPOD), National Institute of Advanced Industrial Science and Technology, Tsukuba Central 6, 1-1-1 Higashi, Tsukuba-city, Ibaraki, 305-8566 Japan, and the accession number of FERM BP-10785 has been assigned. Its mycological properties are as follows.
1. Colony morphology (30°C, 24 hours)
   (1) Cellular morphology: rod-shaped, 0.7 to 0.8 × 1.5 to 2.0 µm
   (2) Gram staining property: negative
   (3) Presence or absence of spore: absence
   (4) Presence or absence of mobility: presence
2. Colony morphology on Nutrient Agar
   Color of colony: pale yellow
   Shape of colony: circular
   Periphery of colony: entire periphery smooth
   Prominence of colony: lens-like
   Transparency: opaque
3. Physiological properties
   (1) Catalase: positive
   (2) Oxidase: negative
   (3) OF test: positive/negative
4. Nucleotide sequence of DNA encoding 16S ribosomal RNA

About 500 bp of a nucleotide sequence of 16S ribosomal DNA was amplified by PCR from the *Stenotrophomonas* sp. SC-1 strain, and the nucleotide sequence was analyzed. Using the resulting nucleotide sequence of the 16S ribosomal DNA, homology retrieval by BLAST was performed and, as a result, the nucleotide sequence shows the highest homology with 16S ribosomal DNA of the *Stenotrophomonas rhizophila* standard strain, their homology being 99.6%. Also in BLAST retrieval using International Nucleotide Sequence Database, the nucleotide sequence of the 16S ribosomal DNA obtained from the *Stenotrophomonas* sp. SC-1 strain shows the highest homology with 16S ribosomal DNA derived from genus *Stenotrophomonas.*

From the above mycological properties, the bacterium was identified as *Stenotrophomonas* sp.

Selective symmetric reduction of a carbonyl group of the compound represented by the formula (1) can be conducted by using a catalytic amount of cells or treated products thereof of these microorganisms.

Next, methods for preparing the present microorganism will be described.

The present microorganism may be cultured using media for culturing various microorganisms which suitably comprise a carbon source, a nitrogen source, organic salts, inorganic salts, and so on.

Examples of the carbon source contained in the medium include glucose, sucrose, glycerol, starch, organic acids and molasses. Examples of the nitrogen source include yeast extracts, meat extracts, peptone, casamino acids, malt extracts, soybean powder, corn steep liquor, cottonseed powder, dry yeast, ammonium sulfide and sodium nitrate. Examples of the organic acid salt and inorganic acid salt may include sodium chloride, potassium chloride, sodium carbonate, potassium phosphate, dipotassium phosphate, calcium carbonate, ammonium acetate, magnesium sulfate, copper sulfate, zinc sulfate, ferrous sulfate and cobalt chloride.

Examples of methods for culturing include solid culturing and liquid culturing (such as test tube culturing, flask culturing and jar fermenter culturing).

The temperature for culturing and the pH of the culture are not particularly limited as long as the present microorganism grows. For example, the culture temperature may be in the range of from about 15°C to about 45°C, and the pH of the culture may be in the range of from about 4 to about 8. The culture time can be suitably selected according to the condition for culturing, and generally in the range from about 1 day to 7 days.

A cell of the present microorganism can be directly used for the production process of the present invention. Examples of the process comprising directly using a cell of the present microorganism include (1) a method comprising directly using a culture and (2) a method comprising collecting the cells by such as centrifugation of a culture and using the collected cells (wet cells washed with buffer or water as necessary).

Alternatively, a treated product of the cell of the present microorganism may be used for the production process of the present invention. Examples of the treated product of the cell include cells obtained by culturing, followed by treating with an organic solvent (such as acetone and ethanol), lyophilizing or treating with an alkaline; physically or enzymatically disrupted cells; crude enzymes obtained by separation or extraction from those cells; and purified enzymes. Examples of the treated product of the cell also include cells subjected to the above-mentioned treatment and immobilized by a publicly known method. Examples of a method of obtaining the immobilized product include a carrier binding method (a method of adsorbing protein or the like onto an inorganic carrier such as silica gel and a ceramic, cellulose or an ion-exchanged resin), and an encapsulating method (a method of confining protein or the like in a network structure of a polymer such as polyacrylamide, sulfur-containing polysaccharide gel (e.g. carrageenan gel), alginic acid gel and agar gel).

The production process of the present invention is generally performed in the presence of water, and reduced nicotineamide adenine dinucleotide phosphate (hereinafter, referred to as NADPH) or reduced nicotineamide adenine dinucleotide (hereinafter, referred to as NADH). The water used thereupon may be an aqueous buffer solution. Examples of a buffer used in the aqueous buffer solution include an alkali metal salt of phosphoric acid such as sodium phosphate and potassium phosphate, and an alkali metal salt of acetic acid such as sodium acetate and potassium acetate.

Alternatively, the production process of the present invention can be carried out in the presence of water and a hydrophobic organic solvent by additionally using a hydrophobic organic solvent. Examples of the hydrophobic organic solvent used in this case include esters such as ethyl formate, ethyl acetate, propyl acetate, butyl acetate, ethyl propionate and butyl propionate, alcohols such as n-butylalcohol, n-amylalcohol and n-octylalcohol, aromatic hydrocarbons such as benzene, toluene and xylene, ethers such as diethyl ether, diisopropyl ether and methyl t-butyl ether, halogenated hydrocarbons such as chloroform and 1,2-dichloroethane, and mixtures thereof.

Alternatively, the production process of the present invention can be carried out in the presence of water and an aqueous medium by additionally using a hydrophilic organic solvent. Examples of the hydrophilic organic solvent used in this case include alcohols such as methanol and ethanol, ketones such as acetone, ethers such as dimethoxyethane, tetrahydrofuran and dioxane, and mixtures thereof.

While the production process of the present invention is generally performed in a range of a pH of the aqueous layer of 3 to 10, it can be suitably changed in such a range that the reaction proceeds.

While the production process of the present invention is generally performed in a range of about 0°C to about 60°C, it can be suitably changed in such a range that the reaction proceeds.

The production process of the present invention is generally performed in a range of for about 0.5 hour to about 10 days. The end point of the reaction can be checked after completion of addition of the carbonyl compound, which is a starting material, by measuring the amount of the carbonyl compound in the reaction solution by liquid chromatography, gas chromatography, and the like.

The concentration of the carbonyl compound, which is the starting material for the production process of the present invention, is generally 50% (w/v) or less. In order to keep the concentration of the carbonyl compound approximately constant in the reaction system, the carbonyl compound may be added to the reaction system continuously or successively.

In the production process of the present invention, if necessary, a sugar such as glucose, sucrose and fructose, or a surfactant such as Triton X-100 and Tween 60 may be added to the reaction system.

After the reaction is completed, the reaction solution is subjected to a general post-treatment such as organic solvent extraction and concentration, whereby the alcohol corresponding to the carbonyl compound may be collected from the reaction solution. The thus-collected alcohol may be further purified by column chromatography, evaporation, and the like, if necessary.

The production process of the present invention is generally carried out in the presence of NADPH or NADH. With the progress of the reductive reaction of a carbonyl compound as a starting material in the production process of the present invention, NADH or NADH is converted into an oxidized form β-nicotinamide adenine dinucleotide phosphoric acid (hereinafter referred to as NADP⁺) or an oxidized form β-nicotinamide adenine dinucleotide (hereinafter referred to as NAD⁺). Since NADP⁺ or NAD⁺ produced by the conversion can be returned back to the original NADPH or NADH with a protein having an ability of converting them into a reduced form (NADPH or NADH), the protein having the ability of converting NADP⁺ or NAD⁺ into NADPH or NADH may be allowed to coexist in the reaction system of the above method.

Examples of the protein having the ability of converting NADP⁺ or NAD⁺ into NADPH or NADH include a glucose dehydrogenase, an alcohol dehydrogenase, an aldehyde dehydrogenase (malic acid dehydrogenase, etc.).

The glucose dehydrogenase as the protein having the ability of converting NADP⁺ or NAD⁺ into NADPH or NADH requires glucose as a substrate in converting NADP⁺ or NAD⁺ into NADPH or NADH, and may be a protein having an ability of converting glucose into gluconolactone through oxidation, or microorganism capable of expressing the protein or a treated product thereof.

In addition, when the protein having the ability to convert NADP⁺ or NADH into NADPH or NADH is a glucose dehydrogenase, activity of the protein is enhanced in some cases by allowing to coexist glucose and the like in a reaction system, and therefore these may be added, for example, to the reaction solution.

In addition, the protein may be an enzyme itself, or may be allowed to coexist in the reaction system in the form of a microorganism having the enzyme or a treated product of the microorganism. Herein, the treated product means an equivalent of the aforementioned "treated product of cell".

### Examples

Hereinafter, the production process of the present invention will be described in detail by way of Examples, but the production process of the present invention is not limited to those Examples.

### Example 1 (Production example of optically-active 2-(2-methylphenyl)-N-methyl-2-hydroxy-acetamide from 2-(2-methyl-phenyl)-N-methyl-2-oxo-acetamide according to the production process of the present invention)

### (1) Synthesis of ethyl 2-(2-methyl-phenyl)-2-oxoacetate

To a mixture of 50 g of tatrahydrofuran and 7.3 g of magnesium was added 4.4 g of 2-bromotoluene to activate the magnesium. A temperature was raised to 40°C, and a solution of 45 g of 2-bromotoluene in tetrahydrofuran (130g) was added dropwise until the disappearance of 2-bromotoluene was observed. Thereafter, the mixture was cooled to room temperature to prepare a Grignard reagent.

A solution of 83 g of diethyl oxalate in toluene (170 g) was cooled to -40°C or lower. The Grignard reagent prepared as described above was added dropwise at -40°C or lower, and this was retained at -40°C for 2 hours. To the reaction mixture was added 234 g of 10% sulfuric acid, and an organic layer was recovered. The resulting organic layer was washed with 134 g of water, and dried with magnesium sulfate. After concentration with an evaporator, distillation under high vacuum, and silica gel column purification afforded 33.1 g of ethyl 2-(methyl-phenyl)-2-oxoacetate.
¹H-NMR (300MHz,CDCl₃):δppm:1.41(t,J=7.2Hz,3H), 2.61(s,3H), 4. 39-4.47(m,2H), 7.26-7.70(m,4H)

### (2) Synthesis of 2-(2-methyl-phenyl)-N-methyl-2-oxo-acetamide

The ethyl 2-(2-methyl-phenyl)-2-oxoacetate obtained in (1) of 18.1 g, 72 g of toluene, and 36 g of methanol were mixed, and 23.2 g of a 40% aqueous methylamine solution was added while retaining at 25°C, and the mixture was retained at 25°C for 1 hour. Thereafter, 36.2 g or water was added, and an organic layer was recovered. The layer was washed with 143 g of 5% hydrochloric acid, 360 g of an aqueous saturated sodium bicarbonate solution, and 36 g of water, respectively, and dried with magnesium sulfate. After concentration with an evaporator, silica gel column purification afforded 11.4 g of 2-(2-methyl-phenyl)-N-methyl-2-oxo-acetamide.
¹H-NMR (300MHz,CDCl₃):δppm:2.48(s,3H), 2.96(d,J=5.13Hz,3H), 7.1(6s,1H), 7.25-7.93(m,4H)

### (3) Asymmetrical reduction of 2-(2-methyl-phenyl)-N-methyl-2-oxo-acetamide using microorganism

Test tubes were charged with 4 ml of a sterilized medium (prepared by adding 20 g of glucose, 5 g of polypeptone, 3 g of yeast extract, 3 g of meat extract, 0.2 g of ammonium sulfate, 1 g of potassium dihydrogenphosphate and 0.5 g of magnesium sulfate 7-hydrate to 1 L of water and then adjusting the pH to 7.0), and the cells shown in Table 1 were inoculated into the medium. These were subjected to shaking culturing at 30°C under aerobic condition. After the culturing was completed, the cells were separated by centrifugation (6000 x g, 10 minutes) and then washed with 0.85% sodium chloride solution to obtain wet cells. To a test tube with a screw cap were added 1.5 mg of 2-(2-methyl-phenyl)-N-methyl-2-oxo-acetamide, about 1.0 mg of the above wet cells, 10 mg of NADPH, and 1.5 ml of a 100 mM phosphate buffer solution (pH 7), and these were shook at 30°C for 23 hours. To the reaction solution, 5 ml of ethyl acetate was added and this was centrifuged (1000 × g, 5 minutes) to recover an organic layer. The organic layer was distilled off to obtain oily 2-(2-methylphenyl)-N-methyl-2-hydroxy-acetamide. ¹H-NMR analysis result of the resulting 2-(2-methyl-phenyl)-N-methyl-2-hydroxy-acetamide is shown below.
¹H-NMR(300MHz,CDCl₃) σ2.37(S,3H), 2.79(d,J=4.96Hz,3H), 3.87(1H), 5.15(S,1H), 6.21(S,1H), 7.15-7.26(m,4H)

To the resulting 2-(2-methyl-phenyl)-N-methyl-2-hydroxy-acetamide, acetonitrile was added, and this was subjected to content analysis by liquid chromatography under the following condition. In addition, to the resulting 2-(2-methyl-phenyl)-N-methyl-2-hydroxy-acetamide was added hexane containing 10% 2-propanol, this was subjected to optical purity analysis by liquid chromatography under the following condition. Conversion rate and the result of the optical purity analysis are shown in Table 1.

Racemic 2-(2-methyl-phenyl)-N-methyl-2-hydroxy-acetamide was synthesized by reducing 2-(2-methyl-phenyl)-N-methyl-2-oxo-acetamide with NaBH₄ in methanol.

### (Content analysis condition)

Column: SUMIPAX ODS A-212 (5 µm, 6 mmφ x 15 cm)
Mobile phase: 0.1% aqueous trifluoroacetic acid solution as A solution, acetonitrile solution containing 0.1% trufluoroacetic acid as B solution

| Time(min) | A solution(%):B solution(%) |
|---|---|
| 0 | 80:20 |
| 20 | 10:90 |
| 30 | 1:99 |
| 30.1 | 80:20 |

Flow rate: 1.0 ml/min
Column temperature: 40°C
Detection: 290 nm
Elusion time
2-(2-methyl-phenyl)-N-methyl-2-oxo-acetamide: 10.4 minutes

### (Optical isomer analysis condition)

Column: CHIRALPAK OD-H (manufactured by Daicel Chemical Industries)
Mobile phase: hexane/2-propanol=90/10 Flow rate: 0.5 ml/min
Column temperature: 40°C
Detection: 230 nm
Elusion time
2-(2-methyl-phenyl)-N-methyl-2-oxo-acetamide: 16.0 minutes 2-(2-methyl-phenyl)-N-methyl-2-hydroxy-acetamide: 20.1 minutes, 23.3 minutes

**Table 1**

| Name of strain | Conversion rate (%) | Optical purity (%e.e) |
|---|---|---|
| Aquaspirillum itersonii subsp.nipponicum IFO 13615t | 61.8 | 31.6 |
| Xanthomonas campestris IFO 13551 | 100 | 94.1 |
| Xanthomonas maltophilia JCM 1975t | 100 | 100 |
| Curtobacterium albidum JCM 1344t | 100 | 100 |
| Curtobacterium citreum JCM 1345t | 100 | 100 |
| Curtobacterium luteum JCM 1480t | 80.4 | 100 |
| Curtobacterium pusillum JCM 1350t | 72.3 | 100 |
| Flavobacterium flavescens JCM 7456 | 81.7 | 14.9 |
| Sphingomonas paucimobilis IFO 13935t | 100 | 96.9 |
| Sphingomonas paucimobilis JCM 7509 | 100 | 96.7 |
| Sphingomonas paucimobilis JCM 7511 | 100 | 96.5 |
| Sphingomonas paucimobilis JCM 7515 | 100 | 97.3 |
| Sphingomonas paucimobilis JCM 7519 | 100 | 96.7 |
| Sphingomonas parapaucimobilis JCM 7512 | 100 | 100 |
| Sphingomonas parapaucimobilis JCM 7520 | 100 | 100 |
| Sphingomonas sp. JCM 7513 | 99.8 | 59.3 |
| Sphingomonas sp. JCM 7514 | 100 | 100 |
| Stenotrophomonas sp. SC-1 | 100 | 100 |
| Stenotrophomonas rhizophila JCM13333 | 100 | 99.1 |

### Example 2 (Production example of optically-active ethyl 2-(2-methyl-phenyl)-2-hydroxy-acetate from ethyl 2-(2-methylphenyl)-2-oxoacetate according to the production process of the present invention)

A reaction was performed according to the same manner as in Example 1(3) except that ethyl 2-(2-methyl-phenyl)-2-oxoacetate was used as a substrate. As a result, oily ethyl 2-(2-methyl-phenyl)-2-hydroxy-acetate was obtained. ¹H-NMR analysis result of the resulting ethyl 2-(2-methyl-phenyl)-2-hydroxy-acetate is shown below.
¹H-NMR (300MHz,CDCl₃) σ1.21 (t, J=7.2Hz, 3H), 2.43(S,3H), 3.56 (d, J=7.7Hz, 1H), 4.13-4.29(m,2H), 5.35(S,1H), 7.15-7.30(m,4H)

To the resulting ethyl 2-(2-methyl-phenyl)-2-hydroxyacetate, acetonitrile was added, and this was subjected to content analysis by liquid chromatography under the following condition. In addition, to the resulting ethyl 2-(2-methylphenyl)-2-hydroxy-acetate was added hexane containing 10% 2-propanol, this was subjected to optical purity analysis by liquid chromatography under the following condition. Conversion rate and the result of the optical purity analysis are shown in Table 2.

Racemic ethyl 2-(2-methyl-phenyl)-2-hydroxy-acetate was synthesized by reducing ethyl 2-(2-methyl-phenyl)-2-oxoacetate with NaBH₄ in methanol.

### (Content analysis condition)

Column: SUMIPAX ODS A-212 (5 µm, 6 mmφ x 15 cm)
Mobile phase: 0.1% aqueous trifluoroacetic acid solution as A solution, acetonitrile solution containing 0.1% trifluoroacetic acid as B solution

| Time(min) | A solution(%):B solution(%) |
|---|---|
| 0 | 80:20 |
| 20 | 10:90 |
| 30 | 1:99 |
| 30.1 | 80:20 |

Flow rate: 1.0 ml/min
Column temperature: 40°C
Detection: 290 nm
Elusion time
ethyl 2-(2-methyl-phenyl)-2-oxoacetate: 17.1 minutes

### (Optical isomer analysis condition)

Column: CHIRALPAK OD-H (manufactured by Daicel Chemical Industries)
Mobile phase: hexane/2-propanol=90/10
Flow rate: 0.5 ml/min
Column temperature: 40°C
Detection: 230 nm
Elusion time
ethyl 2-(2-methyl-phenyl)-2-oxoacetate: 8.9 minutes ethyl 2-(2-methyl-phenyl)-2-hydroxy-acetate: 11.6 minutes, 13.3 minutes

**Table 2**

| Name of strain | Conversion rate (%) | Optical purity (%e.e) |
|---|---|---|
| Aquaspirillum itersonii subsp.nipponicum IFO 13615t | 100 | 93.3 |
| Xanthomonas campestris IFO 13551 | 100 | 98.3 |
| Xanthomonas maltophilia JCM 1975t | 100 | 98.5 |
| Curtobacterium albidum JCM 1344t | 100 | 100 |
| Curtobacterium citreum JCM 1345t | 100 | 98.7 |
| Curtobacterium luteum JCM 1480t | 100 | 97.6 |
| Curtobacterium pusillum JCM 1350t | 100 | 97.7 |
| Flavobacterium flavescens JCM 7456 | 100 | 90.2 |
| Sphingomonas paucimobilis IFO 13935t | 100 | 94.8 |
| Sphingomonas paucimobilis JCM 7509 | 100 | 89.1 |
| Sphingomonas paucimobilis JCM 7511 | 100 | 90.7 |
| Sphingomonas paucimobilis JCM 7515 | 100 | 58.2 |
| Sphingomonas paucimobilis JCM 7519 | 100 | 91.1 |
| Sphingomonas parapaucimobilis JCM 7512 | 100 | 83.6 |
| Sphingomonas parapaucimobilis JCM 7520 | 100 | 94.1 |
| Sphingomonas sp. JCM 7513 | 100 | 23.2 |
| Sphingomonas sp. JCM 7514 | 100 | 100 |
| Stenotrophomonas sp. SC-1 | 100 | 97.0 |
| Stenotrophomonas rhizophila JCM13333 | 100 | 97.4 |

### Example 3 (Production example of optically-active 2-(2-methoxymethyl-phenyl)-N-methyl-2-hydroxy-acetamide from 2-(2-methoxymethyl-phenyl)-N-methyl-2-oxo-acetamide according to the production process of the present invention)

### (1) Synthesis of ethyl 2-(2-methoxymethyl-phenyl)-2-oxoacetate

A temperature of 59.8 g of a 28% solution of sodium methoxide in methanol was raised to 60°C, and a mixture solution of 70.4 g of o-bromobenzyl bromide and 70.4 g of methanol was added dropwise, and this was retained at 60°C for 2 hours. After the mixture was cooled to room temperature, 211 g of toluene and 211 g of water were added thereto, the mixture was stirred, and layers were separated, and an organic layer was recovered. An aqueous layer was extracted with 211 g of toluene three times, the recovered organic layer and 211 g of water were added, and this was washed, and concentrated to obtain 55.3 g of 1-methoxymethyl-2-bromobenzene.

To a mixture of 46.4 g of tetrahydrofuran and 5.4 g of magnesium was added 4.6 g of 1-methoxymethyl-2-bromobenzene to activate the magnesium. A temperature was raised to 40°C, a solution of 41.7 g of 1-methoxymethyl-2-bromobenzene in tetrahydrofuran (139 g) was added dropwise until the disappearance of 1-methoxymethyl-2-bromobenzene was observed. Thereafter, the mixture was cooled to room temperature to prepare a Grignard agent.

A solution of 64.4 g of diethyl oxalate in toluene (177 g) was cooled to -40°C or lower. The Grignard agent prepared as described above was added dropwise at -40°C or lower, and this was retained at -40°C for 4 hours. To the reaction mixture was added 211 g of 10% sulfuric acid, and an organic layer was recovered. The resulting organic layer was washed with 133 g of water, and dried with magnesium sulfate. After concentration with an evaporator, silica gel column purification and distillation under the high pressure afforded 38.5 g of ethyl 2-(2-methoxymethyl-phenyl)-2-oxoacetate.
¹H-NMR (300MHz,CDCl₃):δppm:1.41(t,J=7.08Hz,3H), 3.44(s,3H), 4.41(q,2H), 4.75(s,2H), 7.55-7.69(m,4H)

### (2)Synthesis of 2-(2-methoxymethyl-phenyl)-N-methyl-2-oxo-acetamide

28.3 g of the ethyl 2-(2-methoxymethyl-phenyl)-2-oxoacetate obtained in (1), 109 g of toluene, and 54.4 g of methanol were mixed, 28.6 g of a 40% aqueous methylamine solution was added while retaining at 25°C, and the mixture was retained at 25°C for 2 hours. Thereafter, 54.4 g of water was added, 80 g of toluene were further added, and this was allowed to stand still, and then an organic layer was recovered. The organic layer was washed with 178 g of 5% hydrochloric acid, 54.4 g of an aqueous saturated sodium bicarbonate solution, and 54.4 g of water, respectively, and concentrated with an evaporator to obtain 12.0 g of 2-(2-methoxymethyl-phenyl)-N-methyl-2-oxo-acetamide.
¹H-NMR (300MHz,CDCl₃):δppm:2.95(d,J=5.14Hz,3H), 3.28(s,3H), 4.66(s,2H), 7.04(s,1H), 7.36-7.72(m,4H)

### (3) Asymmetric reduction of 2- (2-methoxymethyl-phenyl)-N-methyl-2-oxo-acetamide using microorganism

A reaction was performed according to the same manner as in Example 1(3) except that 2-(2-methoxymethyl-phenyl)-N-methyl-2-oxo-acetamide was used as a substrate. As a result, oily 2-(2-methoxymethyl-phenyl)-N-methyl-2-hydroxy-acetamide was obtained. ¹H-NMR analysis result of the resulting 2-(2-methoxymethyl-phenyl)-N-methyl-2-hydroxy-acetamide is shown below.
¹H-NMR (300MHz,CDCl₃) σ2.77 (d,J=4.85Hz,3H), 3.47(S,3H), 4.38(d, J=10.6Hz, 1H), 4.73 (d,J=10.6Hz,1H), 4.81 (S,1H), 5.23 (S,1H), 7.16 (S,1H), 7.26-7.43 (m,4H)

To the resulting 2-(2-methoxymethyl-phenyl)-N-methyl-2-hydroxy-acetamide, acetonitrile was added, and this was subjected to content analysis by liquid chromatography under the following condition. In addition, to the resulting 2-(2-methoxymethyl-phenyl)-N-methyl-2-hydroxy-acetamide was added hexane containing 10% 2-propanol, this was subjected to optical purity analysis by liquid chromatography under the following condition. Conversion rate and the result of the optical purity analysis are shown in Table 3.

Racemic 2-(2-methoxymethyl-phenyl)-N-methyl-2-hydroxy-acetamide was synthesized by reducing 2-(2-methoxymethyl-phenyl)-N-methyl-2-oxo-acetamide with NaBH₄ in methanol.

### (Content analysis condition)

Column: SUMIPAX ODS A-212 (5 µm, 6 mmφ x 15 cm)
Mobile phase: 0.1% aqueous trifluoroacetic acid solution as A solution, acetonitrile solution containing 0.1% trifluoroacetic acid as B solution

| Time(min) | A solution(%):B solution(%) |
|---|---|
| 0 | 80:20 |
| 20 | 10:90 |
| 30 | 1:99 |
| 30.1 | 80:20 |

Flow rats: 1.0 ml/min
Column temperature: 40°C
Detection: 290 nm

### Elusion time

2-(2-methoxymethyl-phenyl)-N-methyl-2-hydroxy-acetamide: 9.2 minutes

### (Optical isomer analysis condition)

Column: CHIRALPAK OD-H (manufactured by Daicel Chemical Industries)
Mobile phase: hexane/2-propanol=90/10
Flow rate: 0.5 ml/min
Column temperature: 40°C
Detection: 230 nm

### Elusion time

2-(2-methoxymethyl-phenyl)-N-methyl-2-oxo-acetamide: 18.5 minutes

2-(2-methoxymethyl-phenyl)-N-methyl-2-hydroxy-acetamide: 20.4 minutes, 21.0 minutes

**Table 3**

| Name of strain | Conversion rate (%) | Optical purity (%e.e) |
|---|---|---|
| Aquaspirillum itersonii subsp.nipponicum IFO 13615t | 68.5 | -100 |
| Xanthomonas campestris IFO 13551 | 100 | 100 |
| Xanthomonas maltophilia JCM 1975t | 99.5 | 100 |
| Curtobacterium albidum JCM 1344t | 99.3 | 100 |
| Curtobacterium citreum JCM 1345t | 87.3 | 100 |
| Curtobacterium luteum JCM 1480t | 64.3 | 100 |
| Curtobacterium pusillum JCM 1350t | 60.8 | 100 |
| Flavobacterium flavescens JCM 7456 | 73.4 | -100 |
| Sphingomonas paucimobilis IFO 13935t | 80.5 | 100 |
| Sphingomonas paucimobilis JCM 7509 | 80.1 | 100 |
| Sphingomonas paucimobilis JCM 7511 | 75.4 | 100 |
| Sphingomonas paucimobilis JCM 7515 | 59.6 | 100 |
| Sphingomonas paucimobilis JCM 7519 | 75.3 | 100 |
| Sphingomonas parapaucimobilis JCM 7512 | 90.6 | 100 |
| Sphingomonas parapaucimobilis JCM 7520 | 88.2 | 100 |
| Sphingomonas sp. JCM 7513 | 70.1 | 100 |
| Sphingomonas sp. JCM 7514 | 93.5 | 100 |
| Stenotrophomonas sp. SC-1 | 100 | 100 |
| Slenotrophomonas rhizophila JCM13333 | 92.8 | 100 |

### Example 4 (Production example of optically-active ethyl 2-(2-methoxymethyl-phenyl)-2-hydroxy-acetate from ethyl 2-(2-methoxymethyl-phenyl)-2-oxoacetate according to the production process of the present invention)

A reaction was performed according to the same manner as in Example 1(3) except that ethyl 2-(2-methoxymethyl-phenyl)-2-oxoacetate was used as a substrate. As a result, oily ethyl 2-(2-methoxymethyl-phenyl)-2-hydroxy-acetate was obtained. ¹H-NMR analysis result of the resulting ethyl 2-(2-methoxymethyl-phenyl)-2-hydroxy-acetate is shown below.
¹H-NMR (300MHz,CDCl₃) σ1.21(t,J=7.09Hz,3H), 3.39(S,3H), 4.16-4.26(m,2H), 4.59(d,J=6.36Hz,2H), 5.39(S,1H), 7.31-7.37(m,4H)

To the resulting ethyl 2-(2-methoxymethyl-phenyl)-2-hydroxy-acetate, acetonitrile was added, and this was subjected to content analysis by liquid chromatography under the following condition. In addition, to the resulting ethyl 2-(2-methoxymethyl-phenyl)-2-hydroxy-acetate was added hexane containing 10% 2-propanol, this was subjected to optical purity analysis by liquid chromatography under the following condition. Conversion rate and the result of the optical purity analysis are shown in Table 4.

Racemic ethyl 2-(2-methoxymethyl-phenyl)-2-hydroxyacetate was synthesized by reducing ethyl 2-(2-methoxymethyl-phenyl)-2-oxoacetate with NaBH₄ in methanol.

### (Content analysis condition)

Column: SUMIPAX ODS A-212 (5 µm, 6 mmφ x 15 cm)
Mobile phase: 0.1% aqueous trifluoroacetic acid solution as A solution, acetonitrile solution containing 0.1% trifluoroacetic acid as B solution

| Time(min) | A solution(%):B solution(%) |
|---|---|
| 0 | 80:20 |
| 20 | 10:90 |
| 30 | 1:99 |
| 30.1 | 80:20 |

Flow rate: 1.0 ml/min
Column temperature: 40°C
Detection: 290 nm

### Elusion time

ethyl 2-(2-methoxymethyl-phenyl)-2-hydroxy-acetate: 15.4 minutes

### (Optical isomer analysis condition)

Column: CHIRALPAK OD-H (manufactured by Daicel Chemical Industries)
Mobile phase: hexane/2-propanol=90/10
Flow rate: 0.5 ml/min
Column temperature: 40°C
Detection: 230 nm

### Elusion time

ethyl 2-(2-methoxymethyl-phenyl)-2-oxoacetate: 9.3 minutes ethyl 2-(2-methoxymethyl-phenyl)-2-hydroxy-acetate: 12.8 minutes, 13.6 minutes

**Table 4**

| Name of strain | Conversion rate (%) | Optical purity (%e.e) |
|---|---|---|
| Aquaspirillum itersonii subsp.nipponicum IFO 13615t | 100 | 77.4 |
| Xanthomonas campestris IFO 13551 | 100 | 99.7 |
| Xanthomonas maltophilia JCM 1975t | 100 | 99.9 |
| Curtobacterium albidum JCM 1344t | 100 | 100 |
| Curtobacterium citreum JCM 1345t | 100 | 100 |
| Curtobacterium luteum JCM 1480t | 100 | 99.6 |
| Curtobacterium pusillum JCM 1350t | 100 | 99.4 |
| Flavobacterium flavescens JCM 7456 | 100 | 97.7 |
| Sphingomonas paucimobilis IFO 13935t | 100 | 98.0 |
| Sphingomonas paucimobilis JCM 7509 | 100 | 97.7 |
| Sphingomonas paucimobilis JCM 7511 | 100 | 97.8 |
| Sphingomonas paucimobilis JCM 7515 | 100 | 97.7 |
| Sphingomonas paucimobilis JCM 7519 | 100 | 97.9 |
| Sphingomonas parapaucimobilis JCM 7512 | 100 | 99.7 |
| Sphingomonas parapaucimobilis JCM 7520 | 100 | 100 |
| Sphingomonas sp. JCM 7513 | 100 | 90.1 |
| Sphingomonas sp. JCM 7514 | 100 | 99.6 |
| Stenotrophomonas sp. SC-1 | 100 | 99.8 |
| Stenotrophomonas rhizophila JCM13333 | 100 | 99.1 |

### INDUSTRIAL APPLICABILITY

According to the present invention, optically-active ortho-substituted mandelic acid compounds can be easily produced.

## Claims

1. A process for producing an optically-active ortho-substituted mandelic acid compound represented by the formula (2): 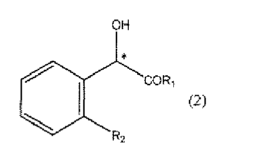 wherein R₁ represents an optionally-substituted amino group or an optionally-substituted alkoxy group, R₂ represents a C1-8 alkyl group optionally substituted with C1-4 alkoxy, and the carbon atom with a symbol * is an asymmetric carbon atom;
the process comprising bringing an ortho-substituted phenylglyoxalic acid compound represented by the formula (1): 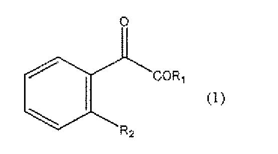 wherein R₁ represents an optionally-substituted amino group or an optionally-substituted alkoxy group, and R₂ represents a C1-8 alkyl group optionally substituted with C1-4 alkoxy;
into contact with a microorganism or a treated product thereof, the microorganism having an ability of reducing the compound of the formula (1) into the optically-active ortho-substituted mandelic acid compound of the formula (2) and being selected from the microorganism group consisting of the genus *Aquaspirillum,* the genus *Xanthomonas,* the genus *Curtobacterium,* the genus *Flavobacterium,* the genus *Sphingomonas* and the genus *Stenotrophomonas.*

2. The process according to claim 1, wherein the microorganism is *Aquaspirillum itersonii, Xanthomonas campestris, Xanthomonas maltophilia, Curtobacterium albidum, Curtobacterium citreum, Curtobacterium luteum, Curtobacterium pusillum, Flavobacterium flavescens, Sphingomonas paucimobilis, Sphingomonas parapaucimobilis, Sphingomonas* sp., *Stenotrophomonas rhizophila,* or *Stenotrophomonas* sp..

3. The process according to claim 1, wherein the microorganism is *Aquaspirillum itersonii* subsp. *nipponicum* IFO 13615t, *Xanthomonas campestris* IFO 13551, *Xanthomonas maltophilia* JCM 1975t, *Curtobacterium albidum* JCM 1344t, *Curtobacterium citreum* JCM 1345t, *Curtobacterium luteum* JCM 1480t, *Curtobacterium pusillum* JCM 1350t, *Flavobacterium flavescens* JCM 7456, *Sphingomonas paucimobilis* IFO 13935t, *Sphingomonas paucimobilis* JCM 7509, *Sphingomonas paucimobilis* JCM 7511, *Sphingomonas paucimobilis* JCM 7515, *Sphingomonas paucimobilis* JCM 7519, *Sphingomonas parapaucimobilis* JCM 7512, *Sphingomonas parapaucimobilis* JCM 7520, *Sphingomonas* sp. JCM 7513, *Sphingomonas* sp. JCM 7514, *Stenotrophomonas rhizophila* JCM13333, or *Stenotrophomonas* sp. SC-1 (FERM BP-10785).
